Europäisches Patentamt

**European Patent Office**

Office européen des brevets

(11) Veröffentlichungsnummer: **0 045 080**
**B1**

(12) **EUROPÄISCHE PATENTSCHRIFT**

(45) Veröffentlichungstag der Patentschrift:
24.10.84

(51) Int. Cl.³: **C 07 D 213/82**

(21) Anmeldenummer: **81105903.9**

(22) Anmeldetag: **25.07.81**

(54) Verfahren zur Reinigung von Nicotinsäureamid II.

(30) Priorität: **30.07.80 DE 3028791**

(43) Veröffentlichungstag der Anmeldung:
**03.02.82 Patentblatt 82/5**

(45) Bekanntmachung des Hinweises auf die Patenterteilung:
**24.10.84 Patentblatt 84/43**

(84) Benannte Vertragsstaaten:
**BE CH DE FR GB IT LI NL**

(56) Entgegenhaltungen:
**DE - A - 2 517 053**
**DE - A - 2 517 054**
**GB - A - 879 551**
**SU - A - 170 509**
**US - A - 2 544 157**
**US - A - 2 932 648**
**US - A - 3 678 060**

*Die Akte enthält technische Angaben, die nach dem*
*Eingang der Anmeldung eingereicht wurden und die*
*nicht in dieser Patentschrift enthalten sind.*

(73) Patentinhaber: **Degussa Aktiengesellschaft,**
**Weissfrauenstrasse 9, D-6000 Frankfurt am Main 1 (DE)**

(72) Erfinder: **Beschke, Helmut, Greifenhagenstrasse 23,**
**D-6450 Hanau 9 (DE)**
Erfinder: **Dahm, Franz-Ludwig, Dipl.-Ing., Kurmainzer**
**Strasse 4, D-8755 Alzenau (DE)**
Erfinder: **Friedrich, Heinz, Dr. Dipl.-Chem.,**
**Grünaustrasse 4, D-6450 Hanau 9 (DE)**
Erfinder: **Prescher, Günter, Dr. Dipl.-Chem.,**
**Liesingstrasse 2, D-6450 Hanau 9 (DE)**

## Beschreibung

Die Erfindung betrifft ein Verfahren zur Gewinnung von reinem Nicotinsäureamid aus rohem, durch alkalische Hydrolyse von Nicotinsäurenitril erzeugtem Nicotinsäureamid. Insbesondere betrifft die Erfindung ein Verfahren zur Befreiung des Nicotinsäureamids von Verunreinigungen durch Nicotinsäure und Salzen der Nicotinsäure.

Nicotinsäureamid wird im allgemeinen durch Hydrolyse von Nicotinsäurenitril in alkalischem Medium erzeugt. Das bei diesen Herstellungsverfahren anfallende rohe Nicotinsäureamid enthält Verunreinigungen, insbesondere Nicotinsäure (im allgemeinen 0,3 bis 5,0%) und Salze der Nicotinsäure (im allgemeinen 1,5 bis 2,5%). Diese Verunreinigungen stören bei der Weiterverwendung des Nicotinsäureamids, namentlich auf pharmazeutischem Gebiet, insbesondere wenn ihr Anteil 0,1% übersteigt.

Es ist bekannt, rohes Nicotinsäureamid mit Hilfe von Ionenaustauschern zu reinigen. Das Nicotinsäureamid wird hierzu als Lösung in Wasser oder polaren organischen Lösungsmitteln, gegebenenfalls bei erhöhter Temperatur bis 50 °C, über einen Anionenaustauscher geführt und es werden hierbei die Nicotinationen gebunden (GB-A-879 551, US-A-3 678 060). Diese Verfahren erfodern erheblichen Aufwand und ergeben schlechte Ausbeuten, wenn ein von Nicotinationen hinreichend gereinigtes Nicotinsäureamid gewonnen werden soll. Überdies sind sie nur für die Fälle geeignet, bei denen eine Abtrennung der Kationen nicht nötig ist.

Es ist auch bekannt, rohes Nicotinsäureamid zu reinigen, indem es in 10%iger wässriger Lösung gleichzeitig mit einem Anionen- und einem Kationenaustauscher behandelt wird (SU-A-170509). Hierdurch wird das Nicotinsäureamid jedoch nicht in dem erfoderlichen Masse von den Verunreinigungen befreit.

Es ist weiterhin bekannt, rohes Nicotinsäureamid durch Umkristallisation zu reinigen. Als Lösungsmittel dienen hierbei Aceton (US-A-2 471 518), Propanol-(2) oder Butylacetat in Gegenwart von Kohle (DE-C-828 247), Äthylacetat (J.Am. Chem. Soc. 65 (1943), 2256 bis 2257), Äthanol in Gegenwart von Kohle (J.Am. Chem. Soc. 70 (1948), 3945), Dioxan oder Petroläther (US-A-2 412 749) oder Benzol (DK-A-87 228). Nachteilig ist bei diesen Verfahren, dass zur Erzielung eines genügend reinen Nicotinsäureamids mehrfache Umkristallisation erforderlich ist und nur eine mässige Ausbeute an reinem Nicotinsäureamid erzielt wird.

Es wurde nun ein Verfahren zur Gewinnung von reinem Nicotinsäureamid aus rohem, durch alkalische Hydrolyse von Nicotinsäurenitril erzeugtem Nicotinsäureamid durch Behandlung einer Lösung des rohen Nicotinsäureamids mit einem sauren und einem basischen Ionenaustauscher gefunden, das dadurch gekennzeichnet ist, dass man das rohe Nicotinsäureamid in 10 bis 18 Gew.-% Wasser enthaltendem 2-Methylpropanol-(1) unter Erwärmen auflöst, die Lösung bei 60 bis 100 °C nacheinander mit dem sauren und dem basischen Ionenaustauscher behandelt, die Lösung abkühlt, das sich abscheidende Nicotinsäureamid abtrennt und die Mutterlauge als Lösungsmittel für weiteres rohes Nicotinsäureamid einsetzt. Bei diesem Verfahren wird bei nur einer einzigen Umkristallisation mit günstiger Ausbeute ein hervorragend reines Nicotinsäureamid gewonnen.

Das erfindungsgemässe Verfahren eignet sich zur Reinigung rohen Nicotinsäureamids, wie es aus den Umsetzungsgemischen gewonnen wird, die bei den üblichen Verfahren zur Herstellung von Nicotinsäureamid bei der Hydrolyse von Nicotinsäurenitril in alkalischem Medium anfallen. Mit Vorteil wird das Verfahren zur Reinigung des nach dem Verfahren gemäss der DE-A-2 517 054 erzeugten Nicotinsäureamids verwendet.

Erfindungsgemäss enthält das 2-Methylpropanol-(1) 10 bis 18 Gewichtsprozent Wasser; insbesondere setzt man ein mit Wasser gesättigtes 2-Methylpropanol-(1), ein.

Zur Durchführung des erfindungsgemässen Verfahrens wird das rohe Nicotinsäureamid im Lösungsmittel unter Erwärmen gelöst. Für die Lösung wird mit Vorteil eine Temperatur von über 50 °C bis nahe dem Siedepunkt der Lösung gewählt, vorzugsweise eine Temperatur von 60 bis 100 °C, insbesondere von 65 bis 85 °C. Es wird zweckmässigerweise eine bei der betreffenden Temperatur möglichst weitgehend gesättigte Lösung bereitet.

Erfindungsgemäss wird die 60 bis 100 °C warme Lösung mit Ionenaustauschern behandelt, und zwar nacheinander in beliebiger Reihenfolge mit einem Kationenaustauscher und einem Anionenaustauscher. Als Kationenaustauscher dient ein handelsüblicher saurer, vorzugsweise ein stark saurer, Ionenaustauscher, beispielsweise auf Polystyrol- oder Polystyrolbenzol-Grundlage, insbesondere ein solcher mit freien Sulfonsäuregruppen, und als Anionenaustauscher wird ein handelsüblicher basischer, vorzugsweise schwach bis mittelstark basischer, Ionenaustauscher, beispielsweise auf Polystyrol- oder Polystyroldivinylbenzol-Grundlage mit makroporöser Struktur, insbesondere ein solcher mit austauschaktiven Aminogruppen, verwendet. Die Behandlung der Nicotinsäureamidlösungen mit den Ionenaustauschern erfolgt in einer beliebigen, für die Durchführung von Ionenaustauschverfahren üblichen Weise. In welcher Menge die Ionenaustauscher angewendet werden, richtet sich nach der Menge Verunreinigungen, die beseitigt werden sollen und nach der gewünschten Reinheit der Lösung.

Nach der Behandlung mit den Ionenaustauschern wird die Lösung abgekühlt und das reine Nicotinsäureamid abgeschieden. Die Mutterlauge wird unmittelbar für einen weiteren Ansatz verwendet.

In dem folgenden Beispiel bedeutet % stets Gewichtsprozent.

**Beispiel**

Es wurden 2500 g rohes Nicotinsäureamid, das 2,3% Natriumnicotinat und 0,8% Nicotinsäure enthielt, mit 2100 g 2-Methylpropanol-(1) und 400 g Wasser vermischt. Die Mischung wurde unter Rückflusskühlung auf Siedetemperatur erhitzt und das Nicotinsäureamid hierbei aufgelöst. Die Lösung wurde dann auf 70 °C abgekühlt und unter Einhaltung dieser Temperatur nacheinander über Säulen eines stark sauren Kationenaustauschers und eines mittelstark basischen Anionenaustauschers geleitet. Die Durchflussgeschwindigkeit betrug 5 Liter Lösung je Liter Austauscher und Stunde. Der Kationenaustauscher war Lewatit S 100 (Austauscher auf Polystyrol-Grundlage mit Sulfonsäuregruppen). Er wurde bis zu einer Kapazität von 1,62 val je Liter Austauscher genutzt und ergab eine Senkung des Gehalts an Natriumionen in der Lösung auf unter 1 ppm, bezogen auf den Gehalt an Nicotinsäureamid in der Lösung. Der Anionenaustauscher war Lewatit MP 64 (Austauscher auf Polystyrol-Grundlage mit Aminogruppen). Er wurde bis zu einer Kapazität von 0,38 val je Liter Austauscher genutzt und bewirkte, dass der Gehalt an Nicotinationen auf unter 0,02%, bezogen auf den Gehalt an Nicotinsäureamid in der Lösung, gesenkt wurde. Danach wurden zur Spülung 250 g 2-Methylpropanol-(1), das 16% Wasser enthielt und auf 70 °C erwärmt war, über die Ionenaustauscher geleitet. Die Spülflüssigkeit wurde mit der übrigen Nicotinsäureamidlösung vereinigt und diese wurde dann langsam auf 10 °C abgekühlt. Das auskristallisierte Nicotinsäureamid wurde abgesaugt, dreimal mit je 150 ml wasserfreiem 2-Methylpropanol-(1) gewaschen und getrocknet. Die Ausbeute betrug 1760 g, entsprechend 73%, bezogen auf das mit der rohen Substanz eingesetzte Nicotinsäureamid. In dem gewonnenen Nicotinsäureamid war Nicotinsäure nicht nachweisbar. Der Natriumgehalt war unter 0,001%. Die nach der Abscheidung des Nicotinsäureamids verbliebene Mutterlauge wurde unmittelbar für weitere Ansätze verwendet. Für die Benutzung bei diesen Ansätzen wurde jeweils der Kationenaustauscher durch Behandlung mit verdünnter wässriger Chlorwasserstoffsäure und der Anionenaustauscher durch Behandlung mit verdünnter wässriger Natriumhydroxidlösung regeneriert. Bei diesen Ansätzen betrug die Ausbeute an Nicotinsäureamid 96%, bezogen auf das mit der rohen Substanz eingesetzte Nicotinsäureamid. Das Nicotinsäureamid wies auch nach 10 Ansätzen die gleiche Reinheit wie das bei dem ersten Ansatz gewonnene Produkt auf.

**Patentanspruch**

Verfahren zur Gewinnung von reinem Nicotinsäureamid aus rohem, durch alkalische Hydrolyse von Nicotinsäurenitril erzeugtem Nicotinsäureamid durch Behandlung einer Lösung des rohen Nicotinsäureamids mit einem sauren und einem basischen Ionenaustauscher, dadurch gekennzeichnet, dass man das rohe Nicotinsäureamid in 10 bis 18 Gew.-% Wasser enthaltendem 2-Methylpropanol-(1) unter Erwärmen auflöst, die Lösung bei 60 bis 100 °C nacheinander mit dem sauren und dem basischen Ionenaustauscher behandelt, die Lösung abkühlt, das sich abscheidende Nicotinsäureamid abtrennt und die Mutterlauge als Lösungsmittel für weiteres rohes Nicotinsäureamid einsetzt.

**Revendication**

Procédé pour la préparation d'amide nicotinique pur à partir d'amide nicotinique brut produit par hydrolyse alcaline de nitrile de l'acide nicotinique, par traitement d'une solution de l'amide nicotinique brut avec un échangeur d'ions acide et un échangeur d'ions basique, procédé caractérisé en ce que l'on dissout l'amide nicotinique brut dans du 2-méthylpropanol-(1) contenant de 10 à 18% en poids d'eau, tout en chauffant, traite la solution à 60 à 100 °C, successivement avec l'échangeur d'ions acide et avec l'échangeur d'ions basique, refroidit la solution, sépare l'amide nicotinique qui se dépose, et utilise la liqueur-mère comme solvant pour une nouvelle quantité d'amide nicotinique brut.

**Claim**

A process for the production of pure nicotinic acid amide from crude nicotinic acid amide which has been produced by the alkaline hydrolysis of nicotinic acid nitrile by treating a solution of crude nicotinic acid amide with an acid and a basic ion exchanger, characterised in that the crude nicotinic acid amide is dissolved with heating in 2-methylpropan-1-ol containing from 10 to 18% by weight of water, the solution is successively treated at from 60 to 100 °C with the acid and the basic ion exchanger, the solution is colled, the precipitating nicotinic acid amide is separated and the mother liquor is used as the solvent for further crude nicotinic acid amide.